(19) Europäisches Patentamt
European Patent Office
Office européen des brevets

(11) **EP 4 251 244 B1**

(12) **EUROPEAN PATENT SPECIFICATION**

(45) Date of publication and mention
of the grant of the patent:
**15.04.2026 Bulletin 2026/16**

(21) Application number: **21807124.9**

(22) Date of filing: **15.11.2021**

(51) International Patent Classification (IPC):
*A61M 5/178* $^{(2006.01)}$     *A61M 5/315* $^{(2006.01)}$
*A61M 5/48* $^{(2006.01)}$      *A61K 31/551* $^{(2006.01)}$

(52) Cooperative Patent Classification (CPC):
**A61M 5/484; A61K 31/551; A61M 5/178;
A61M 5/31513; A61M 5/31515**

(86) International application number:
**PCT/EP2021/081716**

(87) International publication number:
**WO 2022/112043 (02.06.2022 Gazette 2022/22)**

(54) **RATE-LIMITED SYRINGE**

SPRITZE MIT BEGRENZTER RATE

SERINGUE À DÉBIT LIMITÉ

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.11.2020 EP 20210081**

(43) Date of publication of application:
**04.10.2023 Bulletin 2023/40**

(73) Proprietor: **Teva Pharmaceuticals International
GmbH
8645 Jona (CH)**

(72) Inventors:
• **WALDRON, Alexander**
  **Bristol BS1 5BU (GB)**
• **WALLER, Jonathan**
  **Bristol BS1 5BU (GB)**
• **IRIVN, James**
  **Bristol BS1 5BU (GB)**

(74) Representative: **D Young & Co LLP
3 Noble Street
London EC2V 7BQ (GB)**

(56) References cited:
**EP-A1- 2 468 329      US-A- 4 064 879**

**Description**

## TECHNICAL FIELD

The disclosure relates to the field of devices for medicament injection, and more specifically to a syringe and a method of use thereof.

## BACKGROUND

In the medical field, devices such as syringes are well-known for injecting a medicament to a patient. There still remains however a need for improved syringes. For example, injecting certain medicaments at an excessive rate and/or force can result in adverse effects, such as pain, and injection site reactions (ISR) including skin irritation, and skin redness. As a result, there is a need for a manually-operable syringe capable of limiting injection rate and/or force.

US 4 064 879 A discloses a pressure-indicating syringe comprising a barrel, a plunger assembly movable therein and having an outer shaft and an inner piston, finger-engaging elements on the barrel and on the plunger shaft for moving the piston to inject fluid through an apertured end wall of the barrel, and an elastic connection between the finger-engaging elements of the plunger shaft and the piston enabling the piston to be displaced relative to the shaft an amount corresponding to the pressure on the piston during the injection of the fluid, thereby providing an indication of the injection pressure.

EP 2 468 329 A1 discloses an auto injector with a torsion spring.

SUMMARY

It is therefore provided a syringe. The syringe comprises a barrel forming a medicament reservoir therein. The medicament reservoir is capable of fluid communication with a needle hub at a distal end thereof. The syringe further comprises a plunger assembly. The plunger assembly comprises a stopper that seals the medicament reservoir. The stopper is slidable inside the barrel toward the distal end. The plunger assembly further comprises a casing slidable inside the barrel, a distal end of the casing engaging the stopper, a plunger rod and a spring arranged between a distal end of the plunger rod and the stopper.

The plunger assembly further comprises a sleeve engaged with a proximal end of the casing to allow relative translational movement of the sleeve over the casing, and wherein the sleeve is axially fixed to the plunger rod such that distal movement of the sleeve over the casing causes distal movement of the plunger rod inside the casing and relative to the casing. Distal movement of the plunger rod is configured to compress and energize the spring, whereby the spring is configured to expand distally and force the stopper distally through the medicament reservoir in order to expel the medicament through the needle hub. The plunger rod is axially movable between a proximal extremal position and a distal extremal position. The plunger rod is limited from fully compressing the spring throughout an entirety of axial movement from the proximal extremal position to the distal extremal position.

The syringe may comprise one or more of the following:

- a length of the forward axial movement of the plunger rod into the medicament reservoir is variable depending on a length of the forward axial movement of the plunger rod;
- the amount of medicament expelled is controllable by the user;
- the sleeve is prevented from translating distally beyond a proximal end of the medicament reservoir, wherein the syringe further comprises an abutment at the proximal end of the medicament reservoir, the abutment cooperating with an abutting surface of the sleeve to stop forward axial movement of the plunger rod, such that the abutting surface of the sleeve engages the abutment of the syringe in the distal extremal position of the plunger rod
- a length of the forward axial movement of the stopper into the medicament reservoir is proportional to a length of the forward axial movement of the plunger rod;
- a relaxed length of the spring substantially equals a length of the barrel;
- the spring is selected such that a difference between a relaxed length and a fully compressed length of the spring is greater than an axial distance between the proximal extremal position and the distal extremal position of the plunger rod;
- the spring is configured for limiting a rate at which the stopper moves forwardly into the medicament reservoir;
- the plunger rod is configured to be manually moved by a user;
- the length of the forward axial movement of the stopper into the medicament reservoir is proportional to the length of the forward axial movement of the plunger rod
- the casing has a tubular shape and the plunger rod has a tubular shape complementary to the tubular shape of the casing;

- the spring has windings arranged in contact with an inner surface of the casing, the spring being thereby arranged in contact with the inner surface of the casing all along the spring length;
- the distal end of the spring abuts the distal end of the casing, and the distal end of the plunger rod abuts the proximal end of the spring;
- the sleeve comprises one or more rails each guiding a respective pin of the casing, the one or more rails and the respective pins being hidden inside the syringe;
- the spring is made of metal;
- the spring has a spring constant that is comprised between 1N/mm and 3N/mm;
- the spring has a preload distance that is comprised between 2.5mm and 5mm;
- the spring is in a relaxed state when the plunger rod is at the proximal extremal position; and/or
- the syringe comprises a finger flange at a proximal end of the barrel.

It is further provided a method of use of the syringe. The method of use comprises providing the barrel and the plunger assembly. The method of use further comprises providing a vial or other suitable container comprising the medicament, i.e. API *per se* or formulated, for example admixed, with a pharmaceutically acceptable excipient. The method of use optionally further comprises reconstituting, suspending or dissolving the medicament in a pharmaceutically acceptable diluent or vehicle. The term "pharmaceutically acceptable" as used herein means suited for standard pharmaceutical applications, and would not give rise to serious adverse events in patients. The diluent or vehicle may be liquid or semisolid, as understood to one with skill in the art. The method of use further comprises sliding the plunger assembly inside the barrel. The method of use further comprises withdrawing the medicament from the vial by inserting a needle of the syringe in the vial and by operating the plunger assembly. The method of use further comprises inserting the needle at injection location. The method of use further comprises pressing the plunger assembly until the plunger assembly abuts an abutment of the barrel and holding still the plunger assembly until the desired dose of the medicament has been expelled. The API or medicament contained in the syringe as described herein can be used for the treatment and/or prophylaxis of many different types of medical disorders.

It is further provided a kit comprising the barrel and the plunger assembly. The kit may optionally comprise the needle and/or a vial containing the medicament. The kit may optionally comprise a pharmaceutically acceptable diluent or vehicle for the medicament. The diluent or vehicle may be presented in a container, for example a vial or syringe. The kit may further comprise user instructions for reconstituting, suspending or dissolving the medicament (active pharmaceutical ingredient (API) powder, for example) in the container. The kit may further comprise user instructions for withdrawal of the medicament from the container. The kit may further comprise user instructions for performing an injection with the syringe. The components of the syringe may be provided assembled in the kit, or unassembled. For example, the spring, the casing, the barrel, the pins, and the sleeve fixed to the plunger rod may be provided unassembled, as separated components, in the kit. In such a case, the kit may comprise assembly instructions for assembling the components. The instructions may read:

- Insert preload spacers and the spring in the casing;
- Slide the sleeve onto the casing;
- Slightly pre-compress the sleeve and carefully insert the one or more pins;
- Insert the casing into the barrel (a small additional force may have to be applied to push past a ridge feature of the barrel which may be designed to prevent the plunger assembly from accidentally being removed);
- Attach the finger flange to the barrel.

## BRIEF DESCRIPTION OF THE DRAWINGS

Embodiments will now be described, by way of non-limiting example, and in reference to the accompanying drawings, where:

- FIG.s 1A to 5B show examples of the syringe;

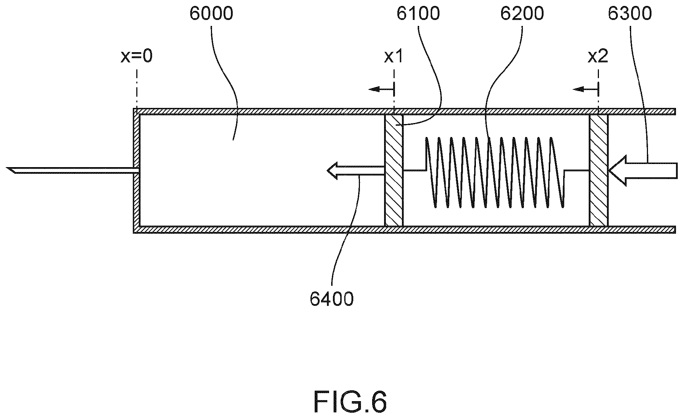

- FIG. 6 represents schematically the functioning of the syringe; and
- FIG.s 7 to 12 represent time evolutions related to injections performed with the syringe.

## DETAILED DESCRIPTION

It is provided a syringe. The syringe comprises a barrel forming a medicament reservoir therein. The medicament reservoir is capable of fluid communication with a needle hub at a distal end thereof. The syringe further comprises a plunger assembly. The plunger assembly comprises a stopper that seals the medicament reservoir and is slidable inside the barrel toward the distal end. The plunger assembly comprises a plunger rod and a spring. The spring is arranged

3

between a distal end of the plunger rod and the stopper. Distal movement of the plunger rod is configured to compress and energize the spring, whereby the spring is configured to expand distally and force the stopper distally through the medicament reservoir in order to expel the medicament through the needle hub. The plunger rod is axially movable between a proximal extremal position and a distal extremal position. The plunger rod being limited from fully compressing the spring throughout an entirety of axial movement from the proximal extremal position to the distal extremal position.

This constitutes an improved syringe.

Indeed, the syringe allows to limit the rate of an injection. Specifically, for performing an injection, a user of the syringe causes axial movement of the plunger rod by applying an injection force on the plunger rod, e.g. by pushing/-pressing the plunger rod, or for example a sleeve of the plunger rod. The user may for example depress the plunger assembly inside the barrel by applying a pressure in the axis of injection. Thereby the speed, force and length of the movement of the plunger rod depend on the force exerted by the user.

If the injection force applied by the user is sufficiently large, then the plunger rod moves axially and forwardly relatively fast and exerts on the proximal end of the spring, upon resistance opposed by the medicament in the reservoir to the forward axial movement of the stopper, a thrust sufficiently large to cause compression of the spring, e.g. the spring constant being sufficiently small. Then, decompression of the spring occurs to slowly and smoothly transmit (with delay) the forward axial movement of the plunger rod to the stopper, thereby slowly and smoothly moving axially and forwardly the stopper in the medicament reservoir. Thereby, the injection is as well slow and smooth.

Now, if the injection force applied by the user is sufficiently small, the plunger rod may move axially and forwardly so as to push the spring, but without compressing the spring, as the force required for compressing the spring may be larger than the thrust of the plunger rod against the spring, e.g. the spring has a sufficiently large spring constant. The spring may thereby move axially and forwardly as the plunger rod, without compression. This movement is converted, since the spring is arranged between the distal end of the plunger rod and the stopper, in a forward axial movement of the stopper in the medicament reservoir. The spring thus transmits (real-time) the forward axial movement of the plunger rod to the stopper. The injection force being small in this case, the injection is still slow and smooth.

Therefore, in both cases, the injection rate is limited: either the user's injection force is sufficiently small to limit the injection rate, either it is not, in which case the spring acts as a regulator to limit the injection rate. In other words, the spring ensures a slow and smooth injection.

The barrel forms a medicament reservoir, i.e. the barrel is a part of the syringe configured for containing a medicament. The inside of the barrel thus forms a cavity which is a reservoir for containing the medicament. For example, the barrel may have a substantially tubular through hole shape. The medicament may comprise or consist of a dissolved and/or suspended medicament. The syringe, or use thereof is not limited by the type of API and the API may be, for example, a small molecule or a biologic drug, per se or admixed with a pharmaceutically acceptable excipient.. The barrel may be partially filled with a medicament or may be totally (i.e. fully) filled with a medicament. At its distal end, the barrel is capable of fluid communication with a needle hub. The barrel may for example comprise, at its distal end, a lumen capable of fluid communication with a lumen of a needle. The distal end of the barrel may be configured for connection of a needle hub of a needle, *e.g.* directly, or indirectly, *e.g.* with the intermediate use of a needle adaptor. The distal end of the barrel may for example form a luer lock adapted for the connection to a needle hub of a needle, e.g. a 3ml luer lock. The syringe may comprise a needle with a needle hub connected to the barrel, optionally with a needle adaptor. At its proximal end, the barrel may comprise a finger flange. The finger flange may form a protrusion on the barrel suited for being gripped by a user. The finger flange may also be referred to as a finger grip. The finger flange helps apply and retain a desired force for injection. The finger flange may be releasably-connected to the barrel, for example attached to it. Alternatively, the finger flange may be integrally-formed with the barrel.

The barrel forms a first component of the syringe. The barrel may be integrally-formed, e.g. injection-molded or 3D-printed. The syringe further comprises a second component formed by the plunger assembly, and thereby including the stopper, the plunger rod and the spring. The two components may be slidable into each other (i.e. telescopic). For example, the second component may be at least partially (e.g. strictly partially or totally) slidable into the barrel. The barrel may comprise a ridge feature configured to prevent the plunger assembly from being accidentally removed from the barrel. Other mechanisms for assembling the two components together may be contemplated. Each component may have a substantially tubular, linear, and straight shape around an axis which may correspond to the direction of the injection force.

The plunger assembly comprises, at its distal end, the stopper. The stopper may thus form a distal part of the plunger assembly. The stopper is slidable inside the barrel and seals the medicament reservoir. For example, the stopper may have a shape complementary to that of the inside of the barrel. For example, the barrel may have a tubular shape, the inside of the barrel forming a tubular hole, and the stopper may have a tubular shape complementary to that of the tubular hole. The stopper may seal the medicament reservoir in that the medicament is contained between the needle hub and the stopper, and can only be expelled through the needle hub, and not in a proximal direction. The stopper may be a bung. The stopper may be made of elastomeric material, *e.g.* rubber, *e.g.* butyl rubber. During injection, when moved axially and forwardly (i.e. distally, in the distal direction), the stopper urges the medicament toward a distal end of the barrel, and through the needle hub.

The amount of medicament thereby expelled may depend on the length of the axial movement of the stopper, that is, the distance traveled by the stopper. The distance travelled by the stopper may be variable depending on a length of the forward axial movement of the plunger rod. In other words, the movement of the plunger rod, which is variable and depends on the user, is converted, by the spring transmitting this movement, into a corresponding forward axial movement of the stopper. The length of the movement of the stopper may depend on the length of the movement of the plunger rod: the longer the length of the movement of the plunger rod is, the longer the length of the movement of the stopper is. If the plunger rod is not axially and forwardly moved, the stopper does not move. If the plunger rod is axially and forwardly moved as far as possible (distally), the stopper is axially and forwardly moved up to the distal end of the medicament reservoir, thereby expelling all the medicament. If the plunger rod is moved up to an intermediate position between these two cases and stopped thereat (the syringe being configured for the plunger rod to be stopped at said intermediate position), then the stopper is moved at a corresponding intermediate position between its initial position (i.e. before movement of the plunger rod) and the distal end of the medicament reservoir (the stopper being then stopped at said corresponding intermediate position). In yet other words, the length of the axial movement of the stopper may be controllable by the user, and may be a function of the length of the forward axial movement of the plunger rod. Thus, the syringe may be configured such that the amount of medicament expelled is controllable by the user.

The length of the forward axial movement of the stopper into the medicament reservoir may be proportional to the length of the forward axial movement of the plunger rod. In other words, the length of the forward axial movement of the plunger rod may be converted by the spring into a proportional length of the forward axial movement of the stopper. Thereby, the volume of medicament expelled is as well proportional to the length of the forward axial movement of the plunger rod, as the volume of medicament expelled is proportional to the length of the forward axial movement of the stopper. For example, the volume of medicament expelled may be equal to the length of the forward axial movement of the stopper multiplied by an elementary volume, e.g. a volume of a section of the medicament reservoir having an elementary length (e.g. 1cm or 1mm). The proportion between the length of the forward axial movement of the stopper into the medicament reservoir and the forward axial movement of the plunger rod may be one-to-one, i.e. the length of the forward axial movement of the stopper into the medicament reservoir may be equal to the length of the forward axial movement of the plunger rod. In other words, the spring may be configured for converting proportionally the length of the forward axial movement of the plunger rod into a length of the forward axial movement of the stopper, and the conversion may be one-to-one.

The stopper is arranged distally relative to the spring, as the spring is arranged between a distal end of the plunger rod and the stopper. In other words, a distal end of the spring is coupled, directly (*e.g.* by a direct contact) or indirectly (*e.g.* by an element arranged between the spring and the stopper), to the stopper. Thereby, the distal axial movement of the stopper is caused by the distal axial movement of the distal end of the spring, e.g. as a result of the decompression of the spring. In an example of indirect coupling between the distal end of the spring and the stopper, the stopper may be coupled (*e.g.* engaged, *e.g.* fixed, *e.g.* snap-fitted) with a component of the plunger assembly housing the spring, for example a casing slidable inside the barrel, and the distal end of the spring may abut a distal end of this component. Thereby, when the distal end of the spring moves axially in the distal direction, e.g. as a result of decompression of the spring, or as a result of the spring transmitting a thrust of the plunger rod without being compressed, as previously discussed, the component moves axially in the distal direction as well, which causes distal axial movement of the stopper, and thereby medicament expulsion.

The spring is arranged distally relative to the distal end of the plunger rod, as the spring is arranged between a distal end of the plunger rod and the stopper. In other words, a proximal end of the spring is coupled, directly (e.g. by a direct contact) or indirectly (e.g. by an element arranged between the spring and the plunger rod), to the distal end of the plunger rod. Thereby, distal axial movement of the plunger rod causes distal axial movement of the proximal end of the spring. The coupling between the proximal end of the spring and the distal end of the plunger rod may consist of an abutment, i.e. the distal end of the plunger rod abuts the proximal end of the spring. Other coupling mechanisms may be contemplated, for example the proximal end of the spring may be attached to the distal end of the plunger rod.

The position of the spring between the distal end of the plunger rod and the stopper allows limitation of the rate of injection by the spring. Upon an injection force exerted by the user, the plunger rod moves axially and distally, and the distal end of the plunger rod pushes the proximal end of the spring in the distal direction. The injection force is thus transformed into a distal thrust exerted by the distal end of the plunger rod against the proximal end of the spring. If this thrust is small enough, the spring is not compressed, and the movement of the plunger rod is transmitted to the spring, and the distal end of the spring transmits the movement to the plunger. If the thrust is large enough, it causes compression of the spring. But as nothing prevents decompression of the spring, decompression occurs, and decompression causes the distal end of the spring to push the plunger axially and distally. Either way, the spring transmits forward axial movement of the plunger rod to the stopper.

The plunger rod is a component of the plunger assembly which is configured for being moved forwardly (*i.e.* distally) and axially upon a user exerting an injection force on the plunger assembly. The plunger rod may have a tubular shape, thereby being slidable into other tubular-shaped components of the syringe. The plunger rod is axially movable between a proximal extremal position and a distal extremal position. The plunger may thus be positioned, upon axial movement, at any position between the proximal extremal position and the distal extremal position. The plunger may remain at said position upon user wish, thereby allowing user control of the quantity of medicament expelled.

The proximal extremal position is the remotest position of the plunger rod with respect to the distal end of the barrel, *i.e.* the distance between the distal end of the barrel and the distal end of the plunger rod is the largest when the plunger rod is in the proximal extremal position. The proximal extremal position is the position of the plunger rod before an injection, that is before a user exerts an injection force on the plunger rod. The spring is in a relaxed state, *i.e.* at rest, *i.e.* substantially uncompressed, when the plunger rod is at the proximal extremal position. By "relaxed state", it is meant that the spring contains no or little pre-loaded energy (e.g. less than 10% of its full energy capacity), such that the spring is incapable by itself to move the stopper. Thereby, the stopper can only be moved by the user exerting a sufficient injection force on the plunger rod that causes the plunger rod to leave the extremal proximal position and move forward toward the distal extremal position. In examples, when the plunger rod is at the proximal extremal position, the spring may present a length equal to its nominal length. In other examples, when the plunger rod is at the proximal extremal position, the spring may present a length differing from its nominal length, by at most 10% from the nominal length. For example, the spring may be partially (but only slightly) compressed.

The distal extremal position is the closest position of the plunger rod with respect to the distal end of the barrel, *i.e.* the distance between the distal end of the barrel and the distal end of the plunger rod is the smallest when the plunger rod is in the distal extremal position. In other words, for performing an injection, a user causes the plunger rod to move axially, thereby leaving the extremal proximal position where the spring is in the relaxed state and moving toward the distal extremal position. This distal axial movement either compresses the spring, either causes it to translate distally, which in both cases, causes distal axial movement of the stopper. In other words, the stopper moves distally axially as the plunger rod moves distally and axially. The total length of the medicament reservoir may be equal to the distance between the proximal extremal position and the distal extremal position. Therefore, if the plunger is moved from the proximal extremal position and up to the distal extremal position, the stopper is moved up to the distal end of the barrel.

The plunger rod may be configured for a forward axial movement at least until a component of the plunger assembly engages a proximal end of the medicament reservoir. For example, the plunger assembly can include a sleeve axially fixed to the plunger rod. The sleeve can be slidable over the casing. In other words, the plunger rod may be configured so as to be allowed to move at least until the sleeve engages a proximal end of the medicament reservoir. This allows the stopper to expel a significant amount of medicament, *e.g.* all the medicament remaining in the barrel.

The plunger rod may be configured for a forward axial movement until the sleeve engages the proximal end of the medicament reservoir. Engagement between the sleeve and the proximal end of the medicament reservoir may thus correspond to the extremal proximal position of the plunger rod. This allows the spring to be fully decompressed/relaxed/at rest after having been pushed by the plunger rod, which helps to obtain a slow and smooth injection. Additionally, the syringe may further comprise an abutment at the proximal end of the medicament reservoir. In such a case, the abutment cooperates with an abutting surface of the sleeve to stop forward axial movement of the plunger rod. This cooperation limits the forward axial movement of the plunger rod. The abutment may form a proximal extremity portion of the barrel.

Further, the plunger rod is limited from fully compressing the spring throughout axial movement of the plunger rod from the proximal extremal position to the distal extremal position. Specifically, engagement between the abutting surface of the sleeve and the abutment of the syringe can limit axial movement of the plunger to prevent full compression of the spring throughout an entirety of movement of the plunger rod. Further, the length of the spring in comparison with the travel distance of the plunger rod between the proximal extremal position and the distal extremal position ensures the plunger rod is incapable of fully compressing the spring throughout an entirety of movement from the proximal extremal position to the distal extremal position.

The spring is a compression spring, arranged between the stopper and the plunger rod in the plunger assembly. The spring may be made of metal. The metal may be a stainless steel. The spring is selected such that a difference between a relaxed length and a fully compressed length of the spring is greater than an axial distance between the proximal extremal position and the distal extremal position of the plunger rod. This prevents axial movement of the plunger rod at any speed from being capable of fully compressing the spring. Were the spring capable of full compression during plunger rod movement, user force could be directly transferred to the stopper at an excessive rate while the spring is in full compression. In contrast, in the present disclosure the configuration of the plunger assembly prevents full compression of the spring at any time, thus ensuring that the spring acts as a constant limit on injection speed, force, and acceleration.

The spring may have a diameter smaller or substantially equal to a diameter of the plunger rod, so that the distal end of the plunger rod abuts the proximal end of the spring. The spring may have a relaxed length, also referred to as "free length", substantially equal to the length of the barrel. This allows expulsion of all the medicament in the medicament reservoir when the medicament reservoir is totally filled with medicament. Alternatively, the spring may have a relaxed length smaller than the length of the medicament reservoir. This allows expulsion of all the medicament in the medicament reservoir when the medicament reservoir is partially filled with medicament.

As stated above, the spring may be configured for limiting a rate at which the stopper moves forwardly into the medicament reservoir. In other words, the spring may be configured to be compressed only when the injection force provided by the user is sufficiently large (*i.e.* above a predetermined threshold). When the force is too small (*i.e.* above said predetermined threshold), then the spring may be not compressed, although the spring still transmits the injection force to the stopper, as previously discussed. Also, the spring may be configured for the decompression to be smooth. In other words, when the injection force provided by the user is large enough, causing the plunger rod to exert a strong thrust on the spring, the spring is first compressed and then decompressed, such that the decompression causes forward distal movement of the stopper smoothly, at a limited rate.

The spring may be configured for a force needed to expel the medicament from the syringe at a rate higher than a predetermined threshold to be higher than a force needed to compress the spring. The force needed to expel the medicament is the force needed so that the stopper can move forwardly and axially in the medicament reservoir, and that this movement urges the medicament through the needle hub. In other words, the force needed to move the stopper distally for causing the expulsion of medicament at high rate, *i.e.* higher than the predetermined threshold, is higher than the force required to compress the spring. Thereby, if the injection force provided by the user is so large that, without spring compression, the stopper would be moved distally at a rate higher than a predefined threshold, then in fact this injection force necessarily compresses the spring. Therefore, the stopper is in fact not moved distally at a rate higher than the predefined threshold and is moved distally smoothly and slowly by the decompression of the spring. The force needed to expel the medicament may include a break loose force, *i.e.* a maximum force required to break the static friction of the stopper in the barrel. The force needed to expel the medicament may alternatively or additionally include a stopper/barrel friction force, *i.e.* a force of friction between an inner surface of the barrel and an outer radial surface of the stopper. The force needed to expel the medicament may additionally or alternatively include hydrodynamic forces, *i.e.* hydrodynamic forces pertaining to the medicament, such as pressure and counter-pressure forces of the stopper pressing the medicament and/or frictional force between the medicament and the barrel (e.g. which depends on viscosity of the medicament and on the shear stress).

The injection force may be provided by the spring being compressed by a user. Specifically, for performing an injection, the user exerts an injection force which drives the plunger rod axially and distally. This injection force is then transmitted to the spring in that the plunger rod transforms this injection force into a thrust exerted on the proximal end of the spring. If the injection force is large enough, the thrust is large enough, thereby causing compression of the spring. The spring then decompresses, which moves the stopper distally in the medicament reservoir, thus providing the injection force to expel the medicament. The user may be a patient in need of the medicament or healthcare professional, for example physician, medic, nurse or physician assistant.

The spring may have a spring constant that is comprised between 1N/mm and 3N/mm. Additionally or alternatively, the spring may have a pre-load distance that is comprised between 2.5mm and 5mm.

For example, the spring may have a spring constant comprised between 2N/mm and 3N/mm, e.g. between 2N/mm and 2.5N/mm, and/or a pre-load distance comprised between 2.5mm and 4mm, e.g. between 2.5mm and 3mm. For example, the spring constant may substantially equal to 2.32N/mm and the pre-load distance may substantially equal 3mm, which results in an injection time/injection rate (e.g. a time for expelling totally the medicament) substantially equal to 10s. In such a case, the maximum force exerted by the spring decompressing during the expulsion may equal 26N, and the force may equal 14N when the piston rod (or the sleeve) bottoms out.

For example, the spring may have a spring constant comprised between 1.5N/mm and 2.5N/mm, *e.g.* between 1.8N/mm and 2.3N/mm, and/or a pre-load distance comprised between 4mm and 5mm, e.g. between 4.2mm and 4.8mm. For example, the spring constant may substantially equal to 1.92N/mm and the pre-load distance may substantially equal 4.5mm, which results in an injection time/injection rate (e.g. a time for expelling totally the medicament) substantially equal to 15s. In such a case, the maximum force exerted by the spring decompressing during the expulsion may equal 19N, and the force may equal 6.5N when the piston rod (or the sleeve) bottoms out.

For example, the spring may have a spring constant comprised between 1N/mm and 2N/mm, *e.g.* between 1,2N/mm and 2N/mm, and/or a pre-load distance comprised between 2mm and 4mm, *e.g.* between 2,5mm and 3.5mm. For example, the spring constant may substantially equal to1.6N/mm and the pre-load distance may substantially equal 3mm, which results in an injection time/injection rate (*e.g.* a time for expelling totally the medicament) substantially equal to 20s. In such a case, the maximum force exerted by the spring decompressing during the expulsion may equal 16N, and the force may equal 4.5N when the piston rod (or the sleeve) bottoms out.

The plunger assembly may include a casing slidable inside the barrel. For example, the casing may have a tubular shape complementary to a tubular shape of the barrel. In such a case, the spring may be arranged inside the casing. The spring mechanism may thus be hidden. For example, the spring may have windings arranged in contact with an inner surface of the casing, the spring being thereby arranged in contact with the inner surface of the casing all along the spring length. For example, the spring may have a diameter complementary to that of the inside of the casing so that the spring windings are in contact with the inner surface of the barrel all along the spring length. The distal end of the casing engages the stopper. For example, the stopper may be fixed, *e.g.* snap-fitted, to the distal end of the casing. The distal end of the spring may abut the distal end of the casing. The distal end of the casing may thus form a surface on which the distal end of the spring abuts on one side, and with which the stopper is engaged on the other side.

The plunger rod is slidable inside the casing. For example, the casing may have a tubular shape, and the rod may have a tubular shape complementary that of the casing. Sliding the rod inside the casing causes the spring either to compress, if the rod is slid with a sufficient distal force, or, if the rod is slid slowly, to transmit the sliding of the rod to the casing, so that the casing slides inside the barrel, because of the abutment of the spring on a distal end of the casing. When the spring is compressed, decompression of the spring and abutment of the spring on a distal end of the casing cause the casing to slide inside the barrel. The distal end of the plunger rod may abut the proximal end of the spring inside the casing, thereby being configured for pushing or compressing the spring.

The plunger assembly further comprises a sleeve axially fixed to the plunger rod and slidable over the casing. The sleeve may be configured to be pushed by a user providing an injection force on the sleeve, *e.g.* by pushing or pressing the sleeve distally. Upon such user action, the sleeve may slide axially and distally over the casing. Since the sleeve is axially fixed to the plunger rod, this axial distal movement of the sleeve over the casing causes the plunger rod to slide distally and axially inside the casing, the plunger rod and the sleeve being for example translationally locked. The sleeve may have a tubular shape substantially complementary to the casing, the plunger rod also having a tubular shape which is complementary to that of the casing. The sleeve may comprise an abutting surface configured to cooperate with an abutment at the proximal end of the barrel upon forward axial movement of the plunger rod. In other words, the abutting surface is configured to abut an abutment formed at the proximal end of the barrel upon forward axial movement of the sleeve with the plunger rod, for example upon a user pushing the sleeve distally. In yet other words, the barrel may comprise an outside abutment cooperating with an abutting surface of the sleeve and/or an inside abutment cooperating with an abutting surface of the plunger rod, the inside and/or the outside abutment allowing to prevent further axial movement of the plunger rod in the distal direction. The fixation between the sleeve and the plunger rod may be of any type. For example, the sleeve and the plunger rod may be two separate parts assembled by any suitable mechanism. Alternatively, the sleeve and the plunger rod may form a single part, *e.g.* which is integrally-formed, *e.g.* injection-molded or 3D-printed.

The sleeve may comprise one or more rails each guiding a respective pin of the casing. This fixes together the sleeve, the plunger rod and the casing. In other words, the casing may comprise one or more pins (*e.g.* brass pins) each protruding into a respective rail of the sleeve, each rail being a groove on the sleeve's surface in which a respective pin is keyed. This stabilizes the plunger assembly. The pins may be integrally-formed with the casing, or, alternatively, be separate parts fixed on the casing. The sleeve may for example comprise two of such rails, for example polar opposite to each other. In such case, the casing may comprise two pins, for example polar opposite to each other. The one or more rails and the respective pins may optionally be hidden inside the syringe. This ensures that the pins are retained. For example, the rails may form grooves on the inner surface of the sleeve, the groove facing the inside of the sleeve, and the pins may protrude outwardly from the casing to engage these grooves, the outward portion of the pins facing the inner surface of the sleeve. This pin/rail mechanism allows higher spring rates and reduces the risk of failures when using the syringe. Also, they reduce friction between the sleeve and the casing. Other mechanisms for arranging the sleeve around the barrel so that the sleeve is engaged with the barrel and slidable over it may be contemplated. For example, the one or more pins may be replaced by snap-fits guided each by a respective rail. The snap-fits may be integrally-formed with the casing (*e.g.* by 3D-printing or injection-molding), or, alternatively, be separate parts fixed on the casing. The sleeve may bottom out, so as to ensure that the spring loads before injection.

The plunger rod, the sleeve, and the casing may be each integrally-formed, *e.g.* 3D-printed or injection-molded.

An example of the syringe is now discussed with reference to the figures.

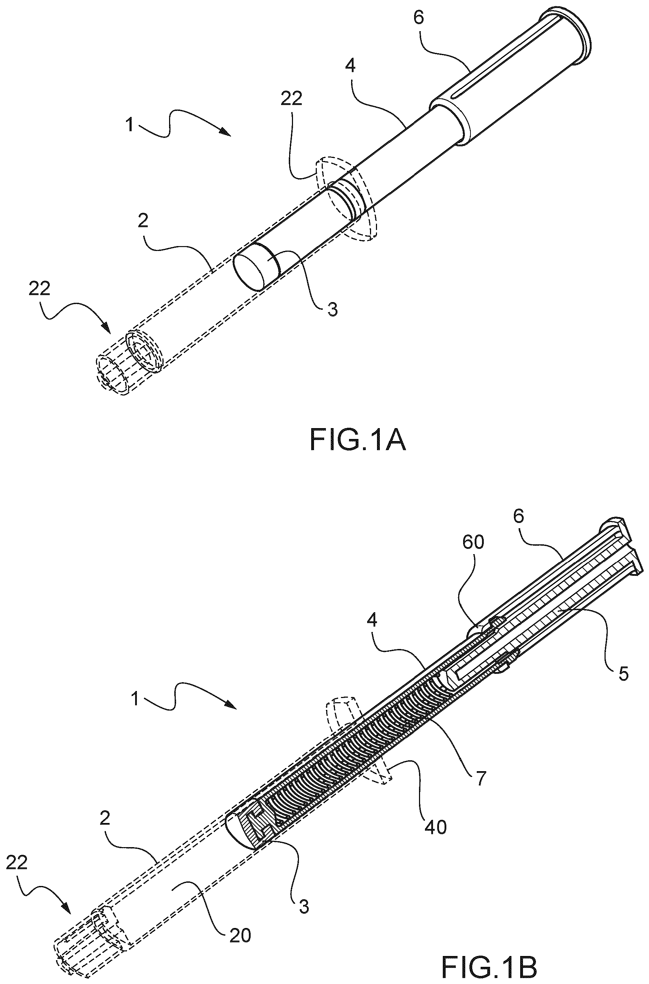

FIG. 1A shows an outside view of a syringe 1 according to this example, and FIG. 1B shows a sectional view B of the syringe 1.

Syringe 1 comprises a barrel 2, forming a medicament reservoir 20 capable at its distal end 22 of fluid communication with a needle hub. At is proximal end, barrel 2 comprises a finger flange 22, configured to be gripped by a user. Barrel 2 has a tubular shape and forms a first component of the syringe 1. At its distal end 22, barrel 2 features a Luer lock configured for connection with a needle hub. In one embodiment, the Luer lock is a 3ml Luer lock, though other embodiments are also contemplated.

Syringe 1 comprises another component slidable in barrel 2. The other component is a plunger assembly formed by stopper/bung 3, casing 4, plunger rod 5, sleeve 6, and spring 7. Casing 4 is slidable inside barrel 2, casing 4 having a tubular shape complementary to that of barrel 2. Stopper 3 is snap-fitted at a distal end of casing 4, stopper 3 having a shape complementary to that of barrel 2, stopper 3 thereby sealing the medicament reservoir 20. Sleeve 6 has a tubular shape complementary to that of casing 4, sleeve 6 being engaged with casing 4 at a proximal end thereof, the engagement allowing relative translational movement of sleeve 6 over casing 4. Sleeve 6 is fixed to plunger rod 5, plunger rod 5 having a tubular shape complementary to that of casing 4, such that distal movement of sleeve 6 over casing 4 causes distal movement of plunger rod 5 inside casing 4.

Spring 7 is arranged inside casing 4, the windings of spring 7 being in contact with casing 4 all along the spring length. At its distal end, spring 7 abuts a distal end portion of casing 4, this distal end portion engaging stopper 3. Spring 7 is arranged between stopper 3 and the distal end of plunger rod 5. The distal end of plunger rod 5 abuts the proximal end of spring 7, thereby providing a direct coupling between the proximal end of spring 7 and the distal end of plunger rod 5.

Spring 7 is configured for limiting a rate at which stopper 3 moves forwardly into the medicament reservoir 20. Spring 7 is configured for a force needed to expel the medicament from syringe 1 at a rate higher than a predetermined threshold to be higher than a force needed to compress spring 7. The force needed to expel the medicament includes at least one of break loose force, stopper/barrel friction force and hydrodynamic forces.

Spring 7 thereby transmits to stopper 3 a forward axial movement of plunger rod 5 upon a user providing an injection force. Specifically, for performing an injection, a user pushes sleeve 6 distally, thereby exerting an injection force on sleeve 6. This triggers forward axial movement of plunger rod 5, the injection force being transmitted to spring 7 by plunger rod 5 abutting the proximal end of spring 7. An abutting surface 60 of sleeve 6 is configured to cooperate with abutment 40 on syringe 1: plunger rod 5 slides distally until abutting surface 60 abuts abutment 40. The abutting surface 60 of the sleeve 6 can comprise the distal end of the sleeve 6, though the present disclosure is not intended to be limited to such. Also, the abutment 40 on syringe 1 can comprise a portion of flange 22. However, it is contemplated that the abutment 40 can be separate from flange 22.

If the injection force is small enough, this thrust does not compress spring 7, and the abutment between the distal end of spring 7 and the distal end of casing 4 transmits the thrust to casing 4, which causes axial distal movement of casing 4 and of stopper 3 engaged with it, at a limited rate. If the injection force is large enough, then the thrust of plunger rod 5 on spring 7 compresses spring 7 until the abutting surface 60 of the sleeve 6 abuts abutment 40 on syringe 1. Decompression of spring 7 then causes axial distal movement of casing 4 and of stopper 3 engaged with it, at a limited rate. Thereby, in this case, the injection force is provided by spring 7 being compressed by the user. In both cases, the injection force is transmitted to stopper 3 from spring 7, where the stopper 3 thereby moves distally and urges the medicament through the needle hub, thereby causing injection.

Medicament reservoir 20 may be filled with a medicament, partially or totally. The amount of medicament expelled depends on a length of the forward axial movement of stopper 3 into medicament reservoir 20, which itself depends on a length of the forward axial movement of plunger rod 5. The user may push sleeve 6 in a controllable manner, that is, up to any desired position between a proximal extremal position and a distal extremal position. As a result, the length of the forward axial movement of stopper 3 into medicament reservoir 20 may be variable and user-controllable, and thereby the amount of medicament expelled may also be variable and user-controllable.

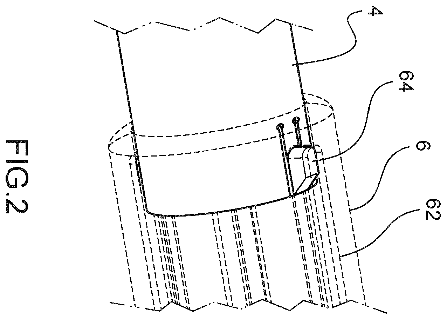

FIG. 2 shows a close-up view of the engagement between casing 4 of syringe 1 and sleeve 6. The engagement is made by rails 62 of casing 4 each engaging a respective snap fit 64 of casing 4. As shown in FIG. 2, snap fits 64 form protrusions on casing 4. On the example shown on FIG. 2, snap fits 64 are integrally-formed with casing 4, e.g. injection-molded.

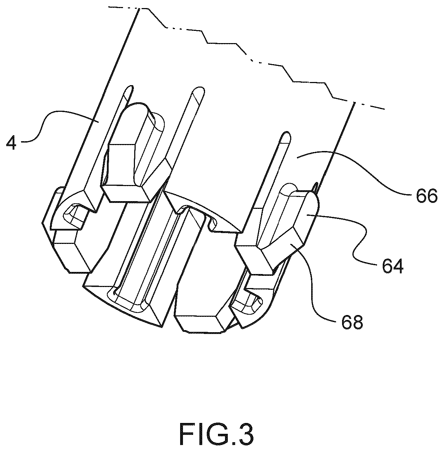

FIG. 3 shows a close-up view of casing 4, with its snap fits 64. As shown on FIG. 3, snap fits 64 form protrusions on casing 4. Specifically, in the example shown on FIG. 3, the proximal end of casing 4 comprises flexible arms 66, the snap fits 64 forming protrusions on flexible arms 66. Snap fits 64 comprise each a sloped surface 68 directed in the proximal direction. Flexible arms 66 and sloped surfaces 68 facilitate assembly of casing 4 with sleeve 6 and plunger rod 5, for example by snap-fitting.

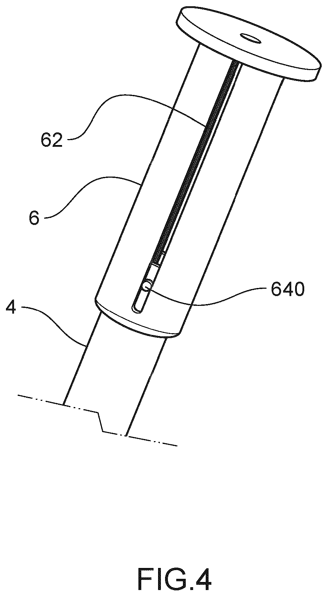

FIG. 4 shows a close-up view of an alternative engagement mechanism between casing 4 of syringe 1 and sleeve 6. The engagement is made by rails 62 of casing 4 each engaging a respective pin 640 of casing 4. Pins 640 replace snap fits 64 of the alternative designs previously shown and are elements of casing 4 protruding out of casing 4. Pins 640 shown on FIG. 4 are not integrally-formed with casing 4, they are separate components mounted on casing 4.

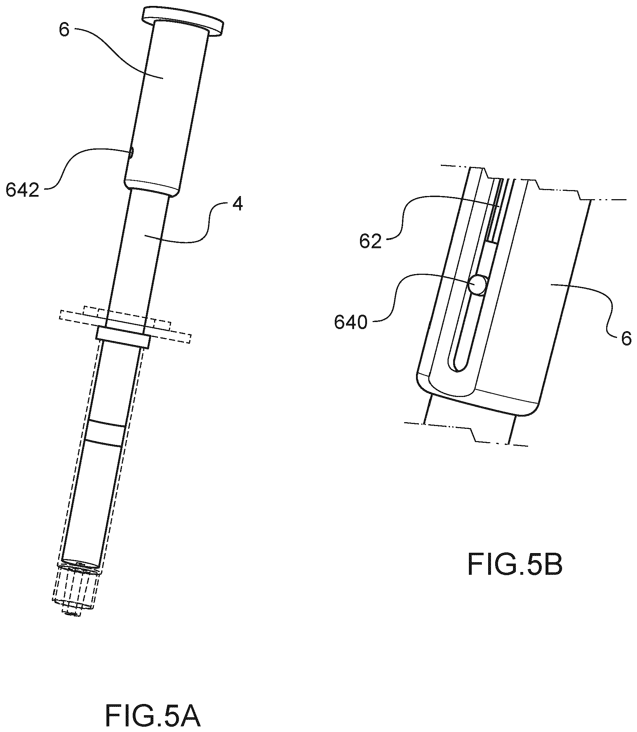

FIG. 5A-5B show yet another alternative design where the pins 640 are hidden. Specifically, rails 62 are formed within the inside/inner surface of sleeve 6, sleeve 6 having a tubular shape. Thereby, rails 62 form grooves on the inner surface of sleeve 6 which face the inside of sleeve 6. Upon assembly of sleeve 6 with casing 4, pins 640 engage rails 62, and the outer surface of pins 640 face the inner surface of sleeve 6 within the grooves formed by rails 62. A close-up inside view of this mechanism is shown on FIG. 5B. As shown, circular openings 642 on the surface of sleeve 6 may indicate an extremity distal position of pins 640 relatively to rails 62. When the position of pins 640, relatively to rails 62, is such that pins 640 cannot slide further distally within rails 62, then the position of pins 640 corresponds to the position of circular openings 642.

The syringe is now further discussed.

As previously said, the syringe allows to limit the rate with which the stopper expels medicament through the needle hub. The syringe may allow dispense rates of 10s, 15s or 20s for a 1.2ml dose of 200 centipoise viscosity suspension. The injection force exerted by the spring to achieve such injection times/rates is now discussed.

Hagen Poiseuille flow may be used as a mathematical model to model the fluid going through the needle hub, this model modelling in general viscous fluid flowing through a long narrow channel such as a needle. Euler's method may be used to discretize this model in time, thereby discretizing the injection process in time. The modeling involves measuring the relative position of the spring measured, to calculate spring force. The force induces a pressure/thrust on the bung/stopper which can be related to the flow rate out of the needle using the Hagen Poiseuille flow equation.

The model is as follows:

$$\Delta_P = \frac{8\mu L Q}{\Pi R^4},$$

where $\mu$ is the fluid viscosity, L is the pipe length, Q is the fluid flow rate, and R is the internal pipe radius.

FIG. 6 represents schematically the functioning of the syringe, including the fluid 6000 in the reservoir formed by the barrel, the stopper 6100, the spring 6200, the thrust 6300 exerted by the plunger rod on the spring 6200, and the decompression force/injection force 6400 exerted by the spring 6200 on the stopper 6100.

The following table shows the defined constants of the modeling (greyed cells may be user-provided).

| Defined Constants | Value | Units | Value | SI units |
|---|---|---|---|---|
| Needle length | 34,73 | mm | 0,03473 | m |
| Barrel inner diameter | 9 | mm | 0,009 | m |
| Needle inner diamater | 0,514 | mm | 0,000514 | m |
| Injection volume | 1,2 | ml | 1,20E-06 | m^3 |
| Dynamic viscosity | 200 | mPa.s | 0,2 | Pa.s |
| Spring constant | 1,22 | N/mm | 1220 | N/m |
| Spring natural length | 50,8 | mm | 0,0508 | m |
| Preload distance | 8 | mm | 0,008 | m |
| Atmospheric pressure | 1 | bar | 101325 | Pa |
| Plunger friction | 0,429 | N | 0,429 | N |
| Dynamic friction | 3 | N | 3 | N |
| Time base | 0,1 | s | 0,1 | s |

The following table shows the calculated constant of the modeling.

| Calculated Constants | Value | Units | Value | SI units |
|---|---|---|---|---|
| Plunger area | 63,61725124 | mm^2 | 6,36173E-05 | m^2 |
| x1 start | 20,00 | mm | 0,020 | m |
| x2 start | 67,83 | mm | 0,06783 | m |

The following table shows the calculated values of the injection volume, injection time, and average spring force.

| Calculated Value | Value | Units |
|---|---|---|
| Total injection volume | 2,51025E-06 | m^3 |
| Injection time | 15 | s |
| Average force | 9,20176094 | N |

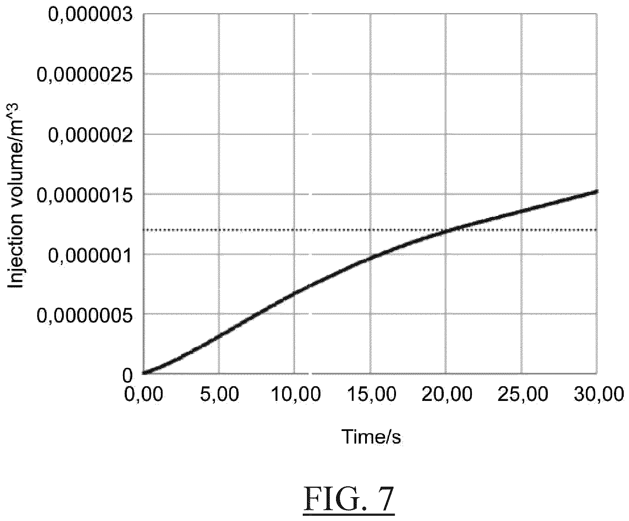

FIG. 7 shows the time evolution of the injection volume for the above constants. As shown in FIG. 7, the injection volume increases relatively slowly over time, illustrating that the spring limits the injection rate.

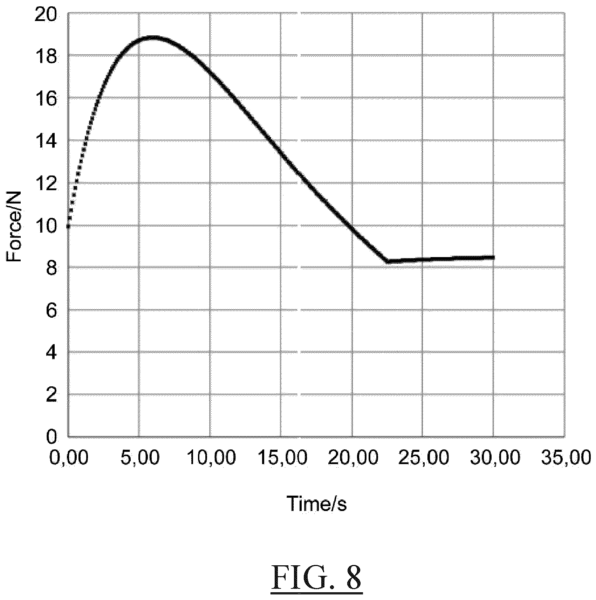

FIG. 8 shows the time evolution of the injection force of the spring over time for the above constants. As shown on FIG. 8, the injection force has a peak and then decreases slowly. This corresponds to the smooth decompression of the spring which thereby limits the injection rate.

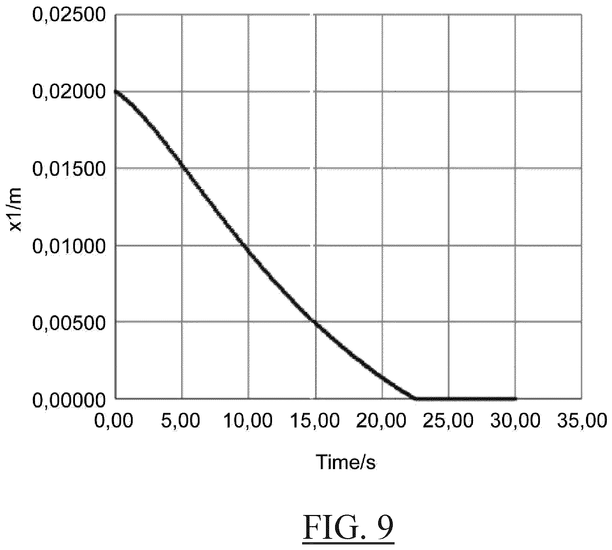

FIG. 9 shows the time evolution of the position x1 of the distal end of the stopper as illustrated on FIG. 6. As shown on FIG. 9, the position x1 smoothly and slowly evolves toward the position x=0 shown in FIG. 6, illustrating the stopper moving slowly and smoothly toward the distal end of the barrel, thereby limiting the injection rate.

The previously discussed modelling shows that an injection time of about 10s can be obtained from a spring with a spring constant of 2.32N/mm and with a pre-load distance of 3mm. The maximum of the spring force is in this case 26N, with a 14N force when the plunger rod bottoms out.

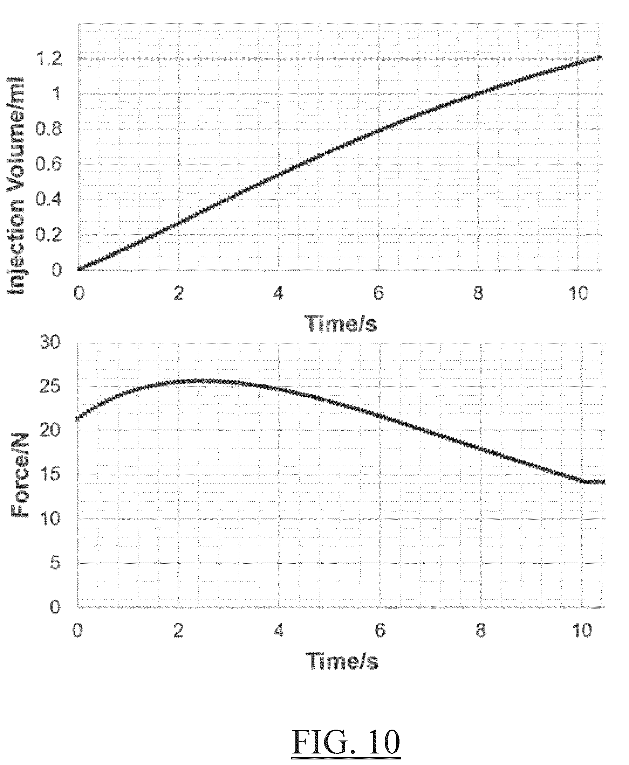

FIG. 10 shows the time evolutions of the injection volume and of the injection force for these parameters of the spring. As shown in FIG. 10, the injection volume increases relatively slowly over time, illustrating how the spring limits the injection rate. The time evolution of the force feature a slight bump and then deceases slowly. This corresponds to the smooth decompression of the spring which thereby limits the injection rate.

The previously discussed modelling shows that an injection time of about 15s can be obtained from a spring with a spring constant of 1.92N/mm and with a pre-load distance of 4.5mm. The maximum of the spring force is in this case 19N, with a 6.5N force when the plunger rod bottoms out.

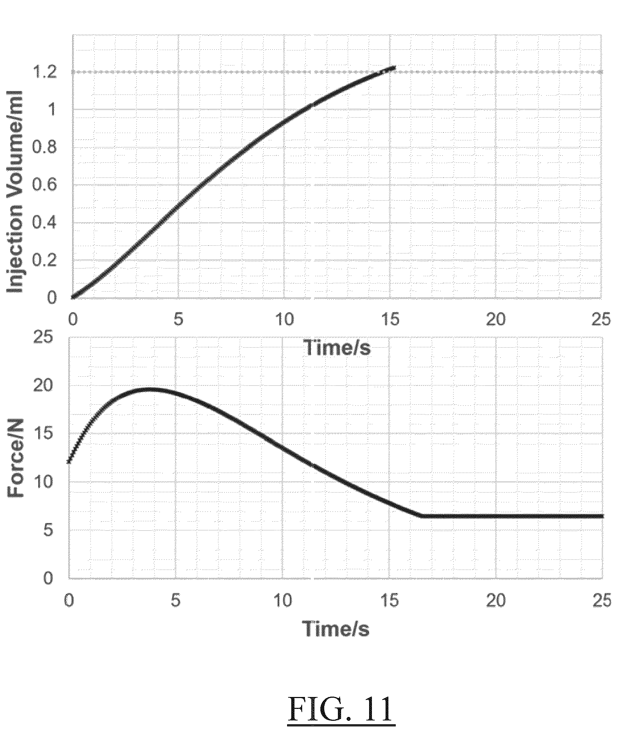

FIG. 11 shows the time evolutions of the injection volume and of the injection force for these parameters of the spring. As shown in FIG. 11, the injection volume increases relatively slowly over time, slower than for the example illustrated by FIG. 10, illustrating how the spring further limits the injection rate in the present example. The time evolution force features a bump and then decreases slowly. This corresponds to the smooth decompression of the spring which thereby limits the injection rate. In the example illustrated on FIG. 11, the bump of the time evolution of the force occurs after that of the example illustrated on FIG. 10, thereby illustrating how the injection time is greater for the example illustrated on FIG. 11.

The previously discussed modelling shows that an injection time of about 20s can be obtained from a spring with a spring constant of 1.6N/mm and with a pre-load distance of 3mm. The maximum of the spring force is in this case 16N, with a 4.5N force when the plunger rod bottoms out.

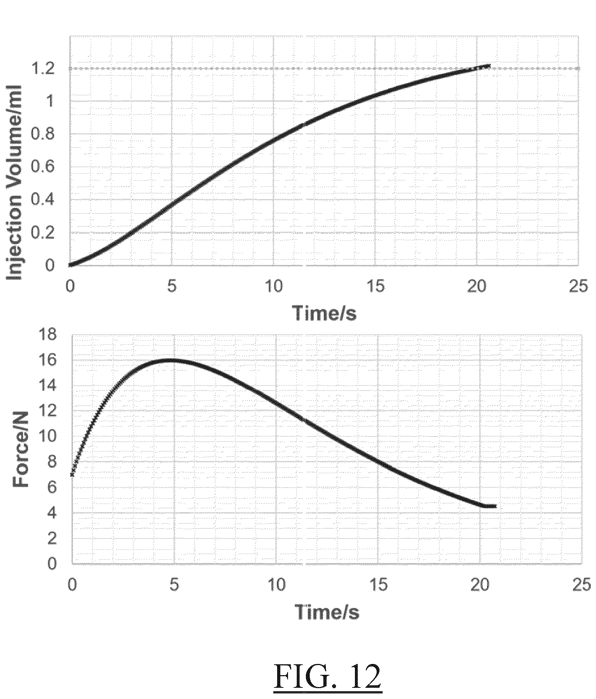

FIG. 12 shows the time evolutions of the injection volume and of the injection force for these parameters of the spring. As shown in FIG. 12, the injection volume increases relatively slowly over time, slower than for the example illustrated by FIG. 11, illustrating how the spring further limits the injection rate in the present example. The time evolution force features a bump and then deceases slowly. This corresponds to the smooth decompression of the spring which thereby limits the injection rate. In the example illustrated on FIG. 12, the bump of the time evolution of the force occurs after that of the example illustrated on FIG. 11, thereby illustrating how the injection time is greater for the example illustrated on FIG. 12.

It is further provided a method of use of the syringe.

The method of use comprises providing the barrel and the plunger assembly, as well as a vial comprising the medicament. For example, the barrel and the plunger assembly may be provided separately, *e.g.* in a kit. The kit may further include the vial. The kit may further include user instructions. The kit may comprise other components, *e.g.* such as a needle comprising a needle hub to connect to the barrel. The method of use may comprise connecting the needle hub to the barrel.

The method of use further comprises sliding the plunger assembly inside the barrel. The method of use further comprises withdrawing the medicament from the vial by inserting a needle of the syringe in the vial and by operating the plunger assembly. For example, a user may grip the plunger assembly, *e.g.* by gripping the sleeve, and pull back the plunger assembly to withdraw medicament from the vial.

The method of use further comprises inserting the needle at injection location, *e.g.* intramuscular, intracutaneous or subcutaneous, of a patient. The method of use further comprises pressing the plunger assembly until the plunger assembly abuts an abutment of the barrel. For example, the user of the syringe may grip the sleeve and press the sleeve distally until the abutment. The method of use further comprises holding still the plunger assembly until all the medicament has been expelled. The method of use may further comprise withdrawing the syringe from the injection location and safely disposing of the needle.

As previously explained, the barrel and the plunger assembly may be included in a kit, optionally comprising the needle and/or a vial containing the medicament. It is further provided such a kit. The kit may further comprise user instructions for performing an injection with the syringe. The components of the syringe may be provided assembled in the kit, or unassembled. For example, the spring, the casing, the barrel, the pins, and the sleeve fixed to the plunger rod may be provided unassembled, as separated components, in the kit. In such a case, the kit may comprise assembly instructions for assembling the components. The instructions may read:

- Insert pre-load spacers and the spring in the casing;
- Slide the sleeve onto the casing;
- Slightly pre-compress the sleeve and carefully insert the one or more pins;
- Insert the casing into the barrel (a small additional force may have to be applied to push past a ridge feature of the barrel which may be designed to prevent the plunger assembly from accidentally being removed);
- Attach the finger flange to the barrel.

**Claims**

1. A syringe (1) comprising:

   a barrel (2) defining a medicament reservoir (20) containing a medicament, the medicament reservoir (20) being in fluid communication with a needle hub at a distal end (22) of the barrel; and
   a plunger assembly comprising:

   a stopper (3) sealing the medicament reservoir and slidable inside the barrel toward the distal end;
   a casing (4) slidable inside the barrel, a distal end of the casing engaging the stopper;
   a plunger rod (5);
   a sleeve (6) engaged with a proximal end of the casing to allow relative translational movement of the sleeve over the casing,
   wherein the sleeve is axially fixed to the plunger rod such that distal movement of the sleeve over the casing causes distal movement of the plunger rod inside the casing and relative to the casing; and
   a spring (7) arranged inside the casing between a distal end of the plunger rod and the stopper,
   wherein distal movement of the plunger rod is configured to compress and energize the spring, whereby the spring is configured to expand distally to force the stopper to move distally through the medicament reservoir to expel the medicament through the needle hub, and
   wherein the plunger rod is axially movable between a proximal extremal position and a distal extremal position, the plunger rod being limited from fully compressing the spring throughout an entirety of axial movement from the proximal extremal position to the distal extremal position.

2. The syringe of claim 1, wherein:

   the sleeve of the plunger assembly is prevented from translating distally beyond a proximal end of the medicament reservoir;
   the syringe further comprises an abutment at the proximal end of the medicament reservoir, the abutment cooperates with an abutting surface of the sleeve to stop distal movement of the plunger rod, such that the abutting surface of the sleeve engages the abutment of the syringe in the distal extremal position of the plunger rod.

3. The syringe of claim 1 or 2, wherein a length of the distal movement of the stopper through the medicament reservoir is proportional to a length of the distal movement of the plunger rod.

4. The syringe of any one of claims 1 to 3, wherein a relaxed length of the spring substantially equals a length of the barrel.

5. The syringe of any of claim 1 to 4, wherein a difference between a relaxed length of the spring and a fully compressed length of the spring is greater than an axial distance between the proximal extremal position and the distal extremal position of the plunger rod.

6. The syringe of any one of claims 1 to 5, wherein the spring is configured to limit a rate at which the stopper moves distally through the medicament reservoir.

7. The syringe of any one of claims 1 to 6, wherein the plunger rod is configured to be manually moved by a user.

8. The syringe of any one of claims 1 to 7, wherein the plunger rod is slidable inside the casing.

9. The syringe of any one of claims 1 to 8, wherein:

   the spring has windings arranged in contact with an inner surface of the casing, the spring being thereby arranged in contact with the inner surface of the casing all along the spring length, and/or
   a distal end of the spring abuts the distal end of the casing, and the distal end of the plunger rod abuts the proximal end of the spring.

10. The syringe of any one of claims 1 to 9, wherein the sleeve comprises one or more rails each guiding a respective pin of the casing.

**11.** The syringe of any one of claims 1 to 10, wherein:

the spring is made of metal,
the spring has a spring constant between 1 N/mm and 3 N/mm, and/or
the spring has a pre-load distance between 2.5 mm and 5 mm.

**12.** The syringe of any one of claims 1 to 11, wherein the plunger assembly is configured such that the spring is in a relaxed state when the plunger rod is at the proximal extremal position.

**13.** The syringe of any one of claims 1 to 12, wherein the syringe comprises a finger flange at a proximal end of the barrel.


**Patentansprüche**

**1.** Spritze (1), umfassend:

einen Zylinder (2), der ein Medikamentenreservoir (20) definiert, das ein Medikament enthält, wobei das Medikamentenreservoir (20) mit einem Nadelansatz an einem distalen Ende (22) des Zylinders in Fluidverbindung steht, und
eine Stößelbaugruppe, die Folgendes umfasst:

einen Stopfen (3), der das Medikamentenreservoir abdichtet und in dem Zylinder zu dem distalen Ende hin verschiebbar ist,
ein in dem Zylinder verschiebbares Gehäuse (4), wobei ein distales Ende des Gehäuses den Stopfen in Eingriff nimmt, eine Stößelstange (5),
eine Hülse (6), die mit einem proximalen Ende des Gehäuses in Eingriff steht, um eine translatorische Relativbewegung der Hülse über das Gehäuse zu gestatten, wobei die Hülse axial an der Stößelstange befestigt ist, so dass eine distale Bewegung der Hülse über das Gehäuse eine distale Bewegung der Stößelstange in dem Gehäuse und relativ zu dem Gehäuse veranlasst, und
eine Feder (7), die in dem Gehäuse zwischen einem distalen Ende der Stößelstange und dem Stopfen angeordnet ist,
wobei die distale Bewegung der Stößelstange dazu ausgestaltet ist, die Feder zu zusammenzudrücken und zu spannen, wobei die Feder dazu ausgestaltet ist, sich distal zu expandieren, um den Stopfen zu zwingen, sich distal durch das Medikamentenreservoir zu bewegen, um das Medikament durch den Nadelansatz auszustoßen, und
wobei die Stößelstange axial zwischen einer proximalen Extremposition und einer distalen Extremposition beweglich ist, wobei die Stößelstange dahingehend begrenzt ist, dass die Feder nicht über eine gesamte axiale Bewegung von der proximalen Extremposition zu der distalen Extremposition vollständig zusammengedrückt wird.

**2.** Spritze nach Anspruch 1, wobei:

verhindert wird, dass die Hülse der Stößelbaugruppe distal über ein proximales Ende des Medikamentenreservoirs hinaus translatiert,
die Spritze ferner einen Anschlag an dem proximalen Ende des Medikamentenreservoirs umfasst, wobei der Anschlag mit einer Anschlagfläche der Hülse zusammenwirkt, um eine distale Bewegung der Stößelstange zu stoppen, so dass die Anschlagfläche der Hülse den Anschlag der Spritze in der distalen Extremposition der Stößelstange in Eingriff nimmt.

**3.** Spritze nach Anspruch 1 oder 2, wobei eine Länge der distalen Bewegung des Stopfens durch das Medikamentenreservoir zu einer Länge der distalen Bewegung der Stößelstange proportional ist.

**4.** Spritze nach einem der Ansprüche 1 bis 3, wobei eine entspannte Länge der Feder im Wesentlichen gleich einer Länge des Zylinders ist.

**5.** Spritze nach einem der Ansprüche 1 bis 4, wobei ein Unterschied zwischen einer entspannten Länge der Feder und einer vollständig zusammengedrückten Länge der Feder größer als ein axialer Abstand zwischen der proximalen Extremposition und der distalen Extremposition der Stößelstange ist.

**6.** Spritze nach einem der Ansprüche 1 bis 5, wobei die Feder dazu ausgestaltet ist, eine Rate zu begrenzen, mit der sich der Stopfen distal durch das Medikamentenreservoir bewegt.

**7.** Spritze nach einem der Ansprüche 1 bis 6, wobei die Stößelstange zur manuellen Bewegung durch einen Benutzer ausgestaltet ist.

**8.** Spritze nach einem der Ansprüche 1 bis 7, wobei die Stößelstange in dem Gehäuse verschiebbar ist.

**9.** Spritze nach einem der Ansprüche 1 bis 8, wobei:
die Feder Windungen aufweist, die in Kontakt mit einer Innenfläche des Gehäuses angeordnet sind, wobei die Feder dadurch entlang der gesamten Federlänge in Kontakt mit der Innenfläche des Gehäuses angeordnet ist und/oder ein distales Ende der Feder an dem distalen Ende des Gehäuses anliegt und das distale Ende der Stößelstange an dem proximalen Ende der Feder anliegt.

**10.** Spritze nach einem der Ansprüche 1 bis 9, wobei die Hülse eine oder mehrere Schienen umfasst, die jeweils einen jeweiligen Stift des Gehäuses führen.

**11.** Spritze nach einem der Ansprüche 1 bis 10, wobei:

die Feder aus Metall hergestellt ist,
die Feder eine Federkonstante zwischen 1 N/mm und 3 N/mm hat und/oder
die Feder einen Vorspannweg zwischen 2,5 mm und 5 mm hat.

**12.** Spritze nach einem der Ansprüche 1 bis 11, wobei die Stößelbaugruppe so ausgestaltet ist, dass sich die Feder in einem entspannten Zustand befindet, wenn sich die Stößelstange in der proximalen Extremposition befindet.

**13.** Spritze nach einem der Ansprüche 1 bis 12, wobei die Spritze einen Fingerflansch an einem proximalen Ende des Zylinders umfasst.

## Revendications

**1.** Seringue (1) comprenant :

un cylindre (2) définissant un réservoir de médicament (20) contenant un médicament, le réservoir de médicament (20) étant en communication fluidique avec une embase d'aiguille au niveau d'une extrémité distale (22) du cylindre ; et
un ensemble plongeur comprenant :

un bouchon (3) fermant le réservoir de médicament et pouvant coulisser dans le cylindre vers l'extrémité distale ;
un boîtier (4) coulissant à l'intérieur du cylindre, une extrémité distale du boîtier entrant en prise avec le bouchon ;
une tige de plongeur (5) ;
un manchon (6) en prise avec une extrémité proximale du boîtier pour permettre un mouvement de translation relative du manchon sur le boîtier,
dans lequel le manchon est fixé axialement à la tige de plongeur de telle sorte qu'un mouvement distal du manchon sur le boîtier provoque un mouvement distal de la tige de plongeur à l'intérieur du boîtier et relativement au boîtier ; et
un ressort (7) disposé à l'intérieur du boîtier entre une extrémité distale de la tige de plongeur et le bouchon, dans lequel le mouvement distal de la tige de plongeur est configuré pour comprimer et activer le ressort, moyennant quoi le ressort est configuré pour se dilater distalement pour forcer le bouchon à se déplacer distalement à travers le réservoir de médicament pour expulser le médicament à travers l'embase d'aiguille, et dans lequel la tige de plongeur est mobile axialement entre une position extrême proximale et une position extrême distale, la tige de plongeur étant empêchée de comprimer complètement le ressort pendant tout le mouvement axial de la position extrême proximale à la position extrême distale.

**2.** Seringue selon la revendication 1, dans laquelle :

le manchon de l'ensemble plongeur est empêché se déplacer en translation distalement au-delà d'une extrémité proximale du réservoir de médicament ;

la seringue comprend en outre une butée à l'extrémité proximale du réservoir de médicament, la butée coopère avec une surface de butée du manchon pour arrêter le mouvement distal de la tige de plongeur, de telle sorte que la surface de butée du manchon vient en prise avec la butée de la seringue dans la position extrême distale de la tige de plongeur.

3. Seringue selon la revendication 1 ou 2, dans laquelle une longueur du mouvement distal du bouchon à travers le réservoir de médicament est proportionnelle à une longueur du mouvement distal de la tige de plongeur.

4. Seringue selon l'une quelconque des revendications 1 à 3, dans laquelle une longueur détendue du ressort est sensiblement égale à une longueur du cylindre.

5. Seringue selon l'une quelconque des revendications 1 à 4, dans laquelle une différence entre une longueur détendue du ressort et une longueur entièrement comprimée du ressort est supérieure à une distance axiale entre la position extrême proximale et la position extrême distale de la tige de plongeur.

6. Seringue selon l'une quelconque des revendications 1 à 5, dans laquelle le ressort est configuré pour limiter une vitesse à laquelle le bouchon se déplace distalement à travers le réservoir de médicament.

7. Seringue selon l'une quelconque des revendications 1 à 6, dans laquelle la tige de plongeur est configurée pour être déplacée manuellement par un utilisateur.

8. Seringue selon l'une quelconque des revendications 1 à 7, dans laquelle la tige de plongeur peut coulisser à l'intérieur du boîtier.

9. Seringue selon l'une quelconque des revendications 1 à 8, dans laquelle :

le ressort présente des spires disposées en contact avec une surface interne du boîtier, le ressort étant de ce fait disposé en contact avec la surface interne du boîtier sur toute la longueur du ressort, et/ou une extrémité distale du ressort bute contre l'extrémité distale du boîtier, et l'extrémité distale de la tige de plongeur bute contre l'extrémité proximale du ressort.

10. Seringue selon l'une quelconque des revendications 1 à 9, dans laquelle le manchon comprend un ou plusieurs rails guidant chacun une broche respective du boîtier.

11. Seringue selon l'une quelconque des revendications 1 à 10, dans laquelle :

le ressort est en métal,
le ressort a une constante de ressort comprise entre 1 N/mm et 3 N/mm, et/ou
le ressort a une distance de précharge comprise entre 2,5 mm et 5 mm.

12. Seringue selon l'une quelconque des revendications 1 à 11, dans laquelle l'ensemble plongeur est configuré de telle sorte que le ressort se trouve dans un état détendu lorsque la tige de plongeur se trouve à la position extrême proximale.

13. Seringue selon l'une quelconque des revendications 1 à 12, la seringue comprenant une bride pour doigt à une extrémité proximale du cylindre.

FIG.1A

FIG.1B

FIG.2

4

66

64

68

FIG.3

FIG.4

FIG.5A

FIG.5B

FIG.6

FIG. 7

FIG. 8

FIG. 9

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4064879 A
- EP 2468329 A1